(19) Europäisches Patentamt / European Patent Office / Office européen des brevets

(11) **EP 1 289 416 B1**

(12) **EUROPEAN PATENT SPECIFICATION**

(45) Date of publication and mention
of the grant of the patent:
**07.11.2007 Bulletin 2007/45**

(51) Int Cl.:
**A61B 5/107** (2006.01)    **G06F 19/00** (2006.01)

(21) Application number: **01947064.0**

(22) Date of filing: **06.06.2001**

(86) International application number:
**PCT/CA2001/000841**

(87) International publication number:
**WO 2001/093752 (13.12.2001 Gazette 2001/50)**

(54) **APPARATUS FOR EVALUATING LABOR PROGRESS DURING CHILDBIRTH**

GERÄT ZUM AUSWERTEN VON GEBURTSDATEN WÄHREND DER GEBURT

APPAREIL POUR EVALUER LE DEGRE D'AVANCEMENT DU TRAVAIL PENDANT L'ACCOUCHEMENT

(84) Designated Contracting States:
**AT BE CH CY DE DK ES FI FR GB GR IE IT LI LU MC NL PT SE TR**

(30) Priority: **08.06.2000  CA 2311029**

(43) Date of publication of application:
**12.03.2003  Bulletin 2003/11**

(73) Proprietor: **LMS Medical Systems, Ltd.**
**Montreal,**
**Quebec H4A 3S5 (CA)**

(72) Inventors:
• **HAMILTON, Emily**
  **Verdun, Quebec H3E 1K8 (CA)**
• **BOISCLAIR, Mario**
  **Montreal, Quebec H2L 2W2 (CA)**
• **KIMANANI, Ebi**
  **Beaconsfield, Quebec H9W 5B6 (CA)**
• **BENDAVID, Bruno**
  **Montreal, Quebec H3W 2M9 (CA)**

(74) Representative: **Somnier, Jean-Louis et al**
**Novagraaf Technologies**
**122, rue Edouard Vaillant**
**92593 Levallois Perret Cedex (FR)**

(56) References cited:
**EP-A- 0 384 339**          **WO-A-00/01117**
**WO-A-98/49942**          **US-A- 5 497 317**
**US-A- 5 851 188**

• HAMILTON E ET AL: "INTRAPARTUM PREDICTION OF FETAL STATUS AND ASSESSMENT OF LABOUR PROGRESS" BAILLIERES CLINICAL OBSTETRICS AND GYNAECOLOGY, BAILLIERE TINDALL, LONDON, GB, vol. 8, no. 3, 8 September 1994 (1994-09-08), pages 567-581, XP001034257 ISSN: 0950-3552
• HAMILTON EMILY F ET AL: "Dystocia among women with symptomatic uterine rupture." AMERICAN JOURNAL OF OBSTETRICS AND GYNECOLOGY, vol. 184, no. 4, March 2001 (2001-03), pages 620-624, XP001033646 ISSN: 0002-9378
• HAMILTON ET AL: "An application of real time decision support in obstetrics" PROC. OF THE INTERN. CONF. ON NEURAL NETWORKS AND EXPERT SYSTEMS IN MEDICINE AND HEALTHCARE, PLYMOUTH, UK, 23 - 26 August 1994, pages 446-455, XP001033465
• HAMILTON E ET AL: "A COMPREHENSIVE LABOR SURVEILLANCE SYSTEM" JOURNAL OF PERINATAL MEDICINE, vol. 15, no. SUPPL. 1, 1987, page 144 XP001034403 ISSN: 0300-5577

**Description**

**Field of the Invention**

This invention relates to the field of medical diagnostic devices and systems in the area of clinical obstetrics and more particularly to an apparatus for the assessment of labour progress during childbirth. This invention is applicable in assisting decision making in clinical medicine and can be used to reduce the number of unnecessary caesarean deliveries.

**Background of the Invention**

The labor of childbirth is the process by which uterine contractions cause the fetus and placenta to be expelled from the uterus and birth canal. Rhythmic contractions of the uterine muscle create a force that pushes the fetus against the opening of the uterus, commonly referred to as the cervix. The cervix is a tubular structure that is firm and closed during pregnancy, keeping the baby and membranes protected inside the uterus. At term, the cervix softens and in labor the continuing pressure of the fetus on the cervix causes it to shorten (efface) and to open (dilate) up to 10 centimeters. As the cervix completely effaces and dilates, the contractions and the mother push the baby through the birth canal. The level of descent of the baby through this passage is referred to as station. Contractions are the forces that promote cervical dilatation. Resistance of the cervix and the birth canal are the opposing forces to the contractions. In addition, the resistance of the cervix changes as it becomes more effaced and more dilated.

Commonly, the effacement, the dilatation, the frequency of the contractions and the station are measured clinically during labor and are used by the doctors to determine if the labor is progressing normally. Generally, if the doctor determines that the labor is progressing normally, the delivery is permitted to continue through the birth canal. However, if the doctor determines that the labor is not progressing normally, a cesarean section is effected to complete the delivery.

Cesarean deliveries are associated with maternal morbidity and an increase in the risk of complications during the current and the subsequent pregnancies. Cesarean deliveries are also more expensive than vaginal births.

Due to the very large number of possible combinations of values for the dilation, the effacement, the frequency of the contractions and the station, the evaluation of labor progress is a difficult task for doctors. Unlike most surgical procedures, there is no suitable postoperative confirmation of the preoperative diagnosis that can be used to validate the doctor's decision. By examining outcomes and cesarean rates in various centers, it is likely that a large number of cesarean deliveries may have been unnecessary.

The article by Emily Hamilton and Ebi Kalahi Kimaniani (xPO01034257) in BAILLIERES CLINICAL OBSTETRICS AND GYNAECOLOGY, BAILLIERE TINDALL, LONDON, GB. vol. 8, no. 3, pages 567 - 581, 08-09-1994 titled INTRAPARTUM PREDICTION OF FETAL STATUS AND ASSESSMENT OF LABOUR PROGRESS discloses which computes expected cervical dilatation measurements based on a model making use of a parity status, a previous dilatation, a previous effacement, a previous level of descent and or contraction count, an apparatus for tracking the labor of a patient during childbirth

Consequently, there is a need in the industry for providing an improved apparatus for evaluating labor progress during childbirth such as to reduce the number of unnecessary cesarean deliveries.

**Summary of the Invention**

The invention provides an apparatus for tracking the labor progress of a patient during childbirth. The apparatus includes an input for receiving a group of clinical measurements associated to a patient. The group of clinical measurements include clinical measurement collection during the last pelvic exam. The group of clinical measurements includes data elements indicative of a measurement of a previous dilatation of the cervix of the patient, a contraction count, a previous level of descent of the child, a previous effacement measurement of the cervix, an epidural status and a parity status. The parity status is indicative of either one of a first childbirth for the patient and a childbirth subsequent to a first childbirth. The apparatus also includes a processing unit operative for generating a reference measure indicative of a certain expected dilatation of the cervix allowing to assess the progress of childbirth by using the following formula:

$$x= AA + BB*(1-y) +CC*z + DD*w +EE*v + FF*u$$

where:

x is the certain expected dilatation of the cervix;
y is the epidural status;
z is the previous dilatation of the cervix measurement;
w is the previous effacement measurement of the cervix;
v is the previous level of descent of the child;
u is the contraction count;

and where AA, BB, CC, DD, EE and FF are a set of real numbers conditioned at least in part on the basis of the parity status. A signal indicative of a measurement of the certain expected dilatation of the cervix is released at an output.

In accordance with a specific example of implementation, the formula includes an error estimate data element indicative of a residual value. The processing unit is further operative to derive a range of expected dilatations of the cervix having a normal distribution on the basis of the error estimate.

Continuing the specific example of implementation, the group of clinical measurements includes a data element indicative of a current dilatation of the cervix measurement. The current dilatation of the cervix measurement to derive a ranking data element indicative of a percentile ranking of the current dilatation of the cervix measurement with respect to the range of expected dilatations of the cervix on the basis of the certain expected dilatation of the cervix and the error estimate. A signal indicative of the ranking data element is then released at the output.

The apparatus described here above can be used for assisting in the determination of whether a cesarean delivery is required for the patient. More specifically, the ranking data element is indicative of the performance of the patient with respect to a reference population. For instance, a percentile of 90 means that the performance of this patient is at the level of, or is better than the lowest 90 out of 100 similar patients who delivered vaginally. In other words, this performance is much faster than average. A percentile ranking of 10 means that the performance of this patient is at the level of, or is better than the lowest 10 out of 100 similar patients who delivered vaginally. In other words, this performance is much slower than average. If a patient has a performance below a certain percentile, herein designated as the minimum normal percentile, the doctor may make the decision that a cesarean may be recommended. The minimum normal percentile may be determined by the hospital in which the delivery is taking place or by health standards or the likes. Typical minimum normal percentiles are in the range of 1.5 to 4 but may be higher or lower depending upon the hospital (or health institution policies) and depending on the condition of the patient.

An advantage of the apparatus according to the present invention is that abnormal labor patterns can be more objectively diagnosed thereby potentially reducing the number of unnecessary cesarean deliveries.

The invention also provides a computer readable medium comprising a program element suitable for execution by a computing apparatus for implementing the above-described apparatus.

Other aspects and features of the present invention will become apparent to those ordinarily skilled in the art upon review of the following description of specific embodiments of the invention in conjunction with the accompanying figures.

## Brief Description of the Drawings

Figure 1 is a block diagram of an apparatus for tracking the labor progress of a patient during childbirth in accordance with a specific example of implementation of the present invention;
Figure 2 is a functional block diagram of the processing unit depicted in figure 1;
Figure 3 is a specific example of a graphical representation of the output signal released by the apparatus of figure 1;
Figure 4 is a block diagram of a system including a broker unit for tracking the labor progress of a patient during childbirth ;
Figure 5 is a functional block diagram of the broker unit depicted in figure 4;
Figure 6 is a flow diagram of a process for tracking the labor progress of a patient during childbirth implemented by the system depicted in figure 4;
Figure 7 is a block diagram of an apparatus for tracking the labor progress of a patient during childbirth in accordance with an alternative example of implementation.

## Detailed Description

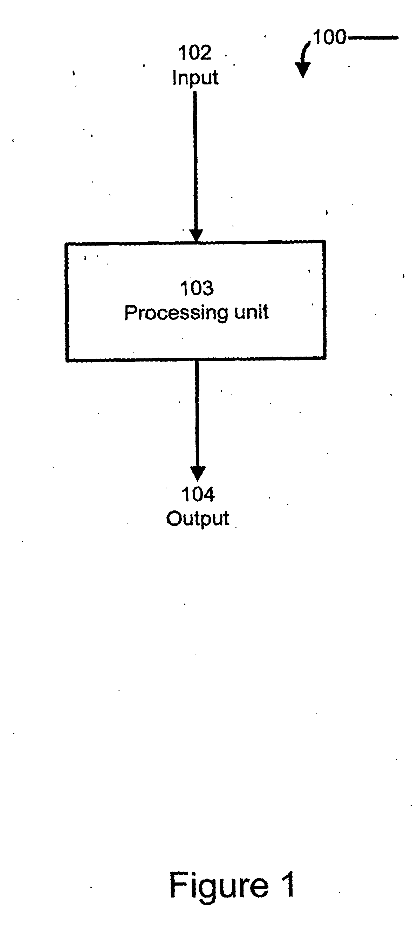

In a specific example of implementation, as shown in figure 1, the invention provides an apparatus 100 for tracking the labor progress of a patient during childbirth. The apparatus 100 includes an input 102, a processing unit 103 and an output 104.

The input 102 is for receiving a group of clinical measurements associated to a patient. The group of clinical measurements includes measurements taken during the last pelvic exam. These measurements will be used to calculate an expected value of the dilatation of the cervix. The group of clinical measurements includes data elements indicative of a measurement of a previous dilatation of the cervix of the patient, a contraction count, a previous level of descent of the child (station), a previous effacement measurement of the cervix, an epidural status and a parity status. More specifically, the clinical measurements are expressed as follows:

- Epidural status =0 if none, =1 if present
- previous dilatation of the cervix measurement = dilatation of the cervix at the previous pelvic examination in centimeters
- previous effacement measurement of the cervix = previous effacement in percent/10
- previous level of descent of the child = level of descent of the child as recorded clinically (-3 to +3) at the previous examination converted to a positive linear scale from 0 to 7. In other words, if the previous level of descent of the child is 1, then the value to be used is 4 (1+3).
- Contraction count is the cumulative contraction count from the time the labor monitoring was started an the first pelvic exam was done. In a specific non-limiting example, contractions are detected algorithmically from a fetal monitor recording and the contraction count is a count of contractions from the time the electronic fetal monitor is applied.

The parity status is indicative of either one of a first childbirth for the patient and childbirth subsequent to a first childbirth. In a non-limiting example, the parity status can take on either one of two values namely a "0" if it is a first childbirth and "1" for other cases. A data element indicative of a current dilatation of the cervix measurement is also received at the input 102.

The output 104 is for releasing a signal indicative of a certain expected dilatation of the cervix. In a specific example of implementation, the output also releases a signal indicative of a band of expected dilatations of the cervix. The expected dilatation of the cervix and the band of expected dilatations are calculated by the processing unit 103 at time intervals on the basis of a sequence of groups of clinical measurements received at the input 102 at these time intervals such as to provide a historical evolution of the patient performance during labor. The result of these calculations is a sequence of expected dilatations of the cervix and bands of expected dilatations of the cervix associated to a sequence of time intervals. The signal indicative of a graphical representation of the sequence of expected dilatations of the cervix and the bands of expected dilatations are released at the output 104. In a very specific example, a curve corresponding to the sequence of expected dilatations of the cervix and ranges of expected dilatations is released at the output 104. The output 104 also releases a signal indicative of a ranking data element. The ranking data element is indicative of a percentile ranking of the current dilatation of the cervix with respect to the calculated certain expected dilatation of the cervix.

In a specific example of implementation, a graphical display unit is coupled to the output 104 such as to provide a visual display of the results. Very specific examples of graphical display units include a computer monitor, a TV monitor, a LCD screen, and a printer among many other suitable display devices.

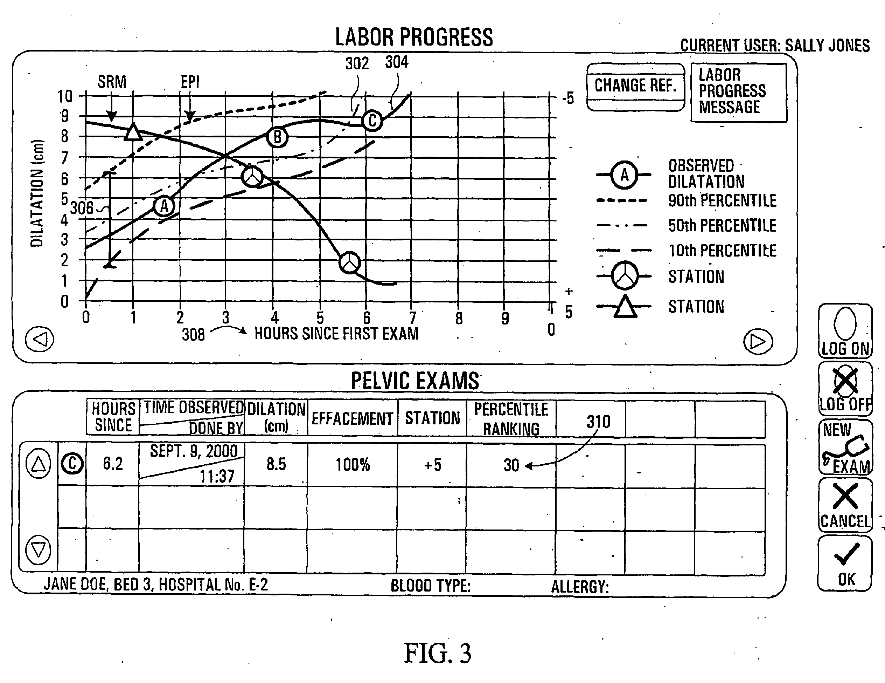

Figure 3 shows a specific example of a graphical representation 350 of the expected dilatation of the cervix 302, the actual dilatation of the cervix 304 and the band of expected dilatations of the cervix 306 as plotted with respect to time 308. The current percentile ranking 310 is also shown.

The processing unit 103 is coupled to the input 102 and the output 104. The processing unit is operative for generating a reference measure indicative of a certain expected dilatation of the cervix allowing assessing the progress of childbirth. A range of expected dilatations of the cervix having a normal distribution is also computed on the basis of an error estimate as well as a ranking data element indicative of a percentile ranking of the current dilatation of the cervix with respect to the calculated expected dilatation. In particular, the ranking data element is released by the apparatus and is used by a doctor for evaluating the progress of labor including assisting in the determination of whether a cesarean delivery is required for the patient.

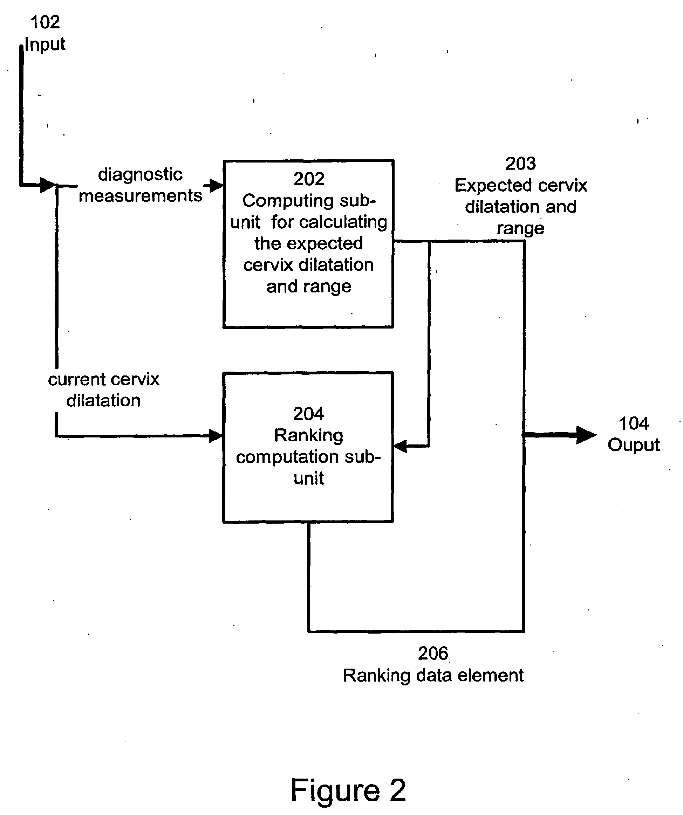

The functionality of the processing unit 103 will be best understood in connection with figure 2 of the drawings. The processing unit can be divided into two sub-units namely a computing sub-unit 202 for calculating the expected cervix dilatation and range of expected cervix dilatations at a certain time and a ranking computation sub-unit 204.

The computing sub-unit 202 generates a reference measure indicative of a certain expected dilatation of the cervix and a range of expected dilatations of the cervix. The computing sub-unit 202 receives as an input the group of clinical measurements namely the previous dilatation of the cervix of the patient, the contraction count, the previous level of descent of the child, the previous effacement measurement of the cervix, the epidural status and the parity status. The group of clinical measurements is processed to compute an expected cervix dilatation and a range of expected cervix dilatations. The computing sub-unit 202 then releases at an output 203 an expected cervix dilatation.

Mathematically, the calculation of the expected dilatation measurement can be expressed by a formula having the form:

## Equation 1

$$x = AA + BB*(1-y) + CC*z + DD*w + EE*v + FF*u$$

where x is the certain expected dilatation of the cervix; y is the epidural status; z is the previous dilatation of the cervix measurement; w is the previous effacement measurement of the cervix; v is the previous level of descent of the child; u is the contraction count; and where AA, BB, CC, DD, EE and FF are a set of real numbers conditioned at least in part on the basis of the parity status.

In accordance with a specific example of implementation, equation 1 includes an error estimate data element indicative of a residual value The error estimate defines a range of expected dilatations of the cervix having a normal distribute about the certain expected dilation of the cervix (which is the mean of the normal distribution).

The error estimate allows calculating a band of expected dilatations of the cervix, the band being defined by a lower boundary and an upper boundary. In a specific example, the band of expected dilatations of the cervix is derived by providing deviation values from the certain expected dilatation of the cervix thereby allowing a band of expected dilatations of the cervix to be defined. Mathematically, the band of expected dilatations of the cervix can be expressed as follows:

## Equation 2

$$Band = [x - z_1R;\ x + z_2R].$$

where x is the certain expected dilatation of the cervix; R is the error estimate and $z_1$ (lower deviation) and $z_2$ (upper deviation) are selected on the basis of a desired area under a curve indicative of the normal distribution of the expected dilations of the cervix. The person skilled in the art will readily observe that the upper deviation and lower deviation may differ from one another. For the purpose of simplicity, it will be assumed that the lower deviation and the upper deviation have the same value and the expression boundary deviation will be used to designate both the lower deviation and the upper deviation. In a specific example, the boundary deviation is a real number.

In a non-limiting example, the computing sub-unit 202 provides two sets of real numbers AA, BB, CC, DD, EE and FF, namely a first set and a second set, each set being associated to a respective parity status. The computing sub-unit 202 processes the parity status data element to select a set of real numbers on the basis of the parity status. In other words, the expected dilatation measurement of a cervix during childbirth is dependent at least in part upon whether the childbirth is a first childbirth (primiparous women) for the patient or a childbirth subsequent to a first childbirth (multiparous women). In addition, the computing sub-unit 202 also provides two error estimate values, namely a first error estimate and a second error estimate, each error estimate being associated to a respective parity status.

More specifically, when the parity status is indicative of a first childbirth, the error estimate has a value of about 1.3 and the set of real numbers is a first set characterized by:

  i. AA having a value of about 0.25 ;
  ii. BB having a value of about 0.15 ;
  iii. CC having a value of about 0.8;
  iv. DD having a value of about 0.24;
  v. EE having a value of about 0.04;
  vi. FF having a value of about 0.004.

A very specific non-limiting example of implementation makes use of a error estimate of 1.3014 and a first set characterized by:

  i. AA having a value of about 0.24952;
  ii. BB having a value of about 0.14627;
  iii. CC having a value of about 0.80515;
  iv. DD having a value of about 0.24109;
  v. EE having a value of about 0.03909;
  vi. FF having a value of about 0.00447.

Continuing the specific example of implementation, when the parity status is indicative of a childbirth subsequent to a first childbirth, the error estimate has a value of about 1.3 and the set of real numbers is a second set characterized by:

> i. AA having a value of about 2;
> ii. BB having a value of about 0.2;
> iii. CC having a value of about 0.8 ;
> iv. DD having a value of about 0.15;
> v. EE having a value of about 0.2;
> vi. FF having a value of about 0.01.

A very specific non-limiting example of implementation makes use of a error estimate of 1.3451 and a second set characterized by:

> i. AA having a value of about 1.9914;
> ii. BB having a value of about 0.23484;
> iii. CC having a value of about 0.7770;
> iv. DD having a value of about 0.15503;
> v. EE having a value of about 0.20669;
> vi. FF having a value of about 0.00766.

The skilled person in the art will readily appreciate that the above coefficient values depend on the units used with respect to the clinical measurements and that conversion values may be applied to the above real numbers to adapt the equation to measuring units different from the ones used in this specific example. For instance, in the examples presented in this description, the previous dilatation of the cervix measurement is measured in centimeters (cm) and therefore the coefficient CC is conditioned on that basis. In order to use a previous dilatation of the cervix measurement measured in inches, the appropriate conversion factor must be applied to the coefficient CC. The same principle applies to the other coefficients.

The expected cervix dilatation can be compared to the actual or observed dilatation in order to determine if the labor is progressing normally or whether a cesarean delivery is required.

The ranking computation sub-unit 204 is coupled to the output of the computing sub-unit 202 and receives as input the expected cervix dilatation and the error estimate computed by the computing unit 202. The ranking computation sub-unit 204 also receives as input a measure of the current dilation of the cervix. The measure of the current dilatation of the cervix is processed by the ranking computation sub-unit 204 to derive a ranking data element indicative of a percentile ranking of the current dilatation of the cervix with respect to the error estimate and the certain expected dilation of the cervix. The ranking data element is then released at an output 206.

Advantageously, the error estimate and the certain expected dilatation of the cervix defined a range of expected dilatations, having a normal distribution thereby allowing the difference between the actual and the expected dilations to be expressed in percentiles. In a specific example of implementation, the percentile ranking is computed on the basis of the assumption that the range of expected dilatations of the cervix is indicative of a normal distribution (bell shaped). Statistical methods can be used to compute the percentile ranking and are well known in the art to which this invention pertains and will not be further described here. As an example, a percentile can be calculated by the following formula:

$$\texttt{Percentile} = \int (\texttt{n}) \ * \ [(\texttt{current\_x} -\texttt{x})/\texttt{R} \ ]$$

Where n is the normal distribution and current_x is the current dilatation measurement of the cervix.

Advantageously, the percentile ranking can be used by a doctor to assess the labor progress of the patient with respect to a reference population. The usual fashion of interpreting percentile rankings can be used. For instance, a percentile of 90 means than the performance of the patient is at the level of, or is better than the lowest 90 out of 100 similar patients who delivered vaginally. In other words, this performance is much faster than average. A percentile ranking of 10 means that the performance of the patient is at the level of, or is better than the lowest 10 of 100 similar patients who delivered vaginally. In other words, this performance is much slower than average. The percentile ranking can be used in assisting the doctor to determine whether a cesarean delivery is recommended. For instance, if a patient has a performance below the minimum normal percentile, the doctor may take the decision that a cesarean is to be recommended. The minimum normal percentile may be determined by the hospital in which the delivery is taking place or by health standards or the likes. Typical minimum normal percentiles are in the range of 1.5 to 4 but may be higher or lower depending upon the hospital (or health institution policies).

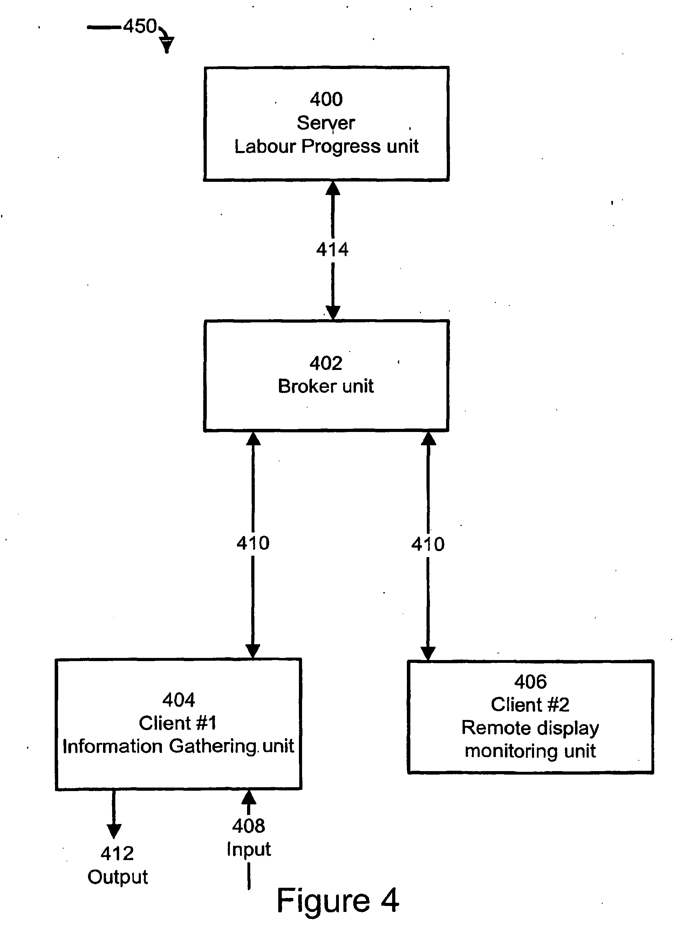

As shown in figure 4, according to an example not forming part of the claimed invention, there is provided a system 450 for tracking the labor progress of a patient during childbirth. In a specific example of implementation, the system makes use of a broker unit network architectural pattern. The system includes a set of components namely server units, a broker unit 402 and a set of client units. A plurality of server units and any number of client units may be present in the system 450. For the sake of simplicity, a system including a single server unit namely the labor progress unit 400 and two client units namely an information gathering unit 404 and a remote display monitoring unit 406 will be described herein below.

In the broker unit network architectural pattern, the components of the network are co-ordinated by the broker unit 402. Each component in the set of components is associated to a respective entity identifier allowing distinguishing a certain component from the other components in the set of components. Components communicate with one another using the entity identifiers in combination with well-known communication protocols such as for example the Transmission Control Protocol/Internet Protocol (TCP/IP).

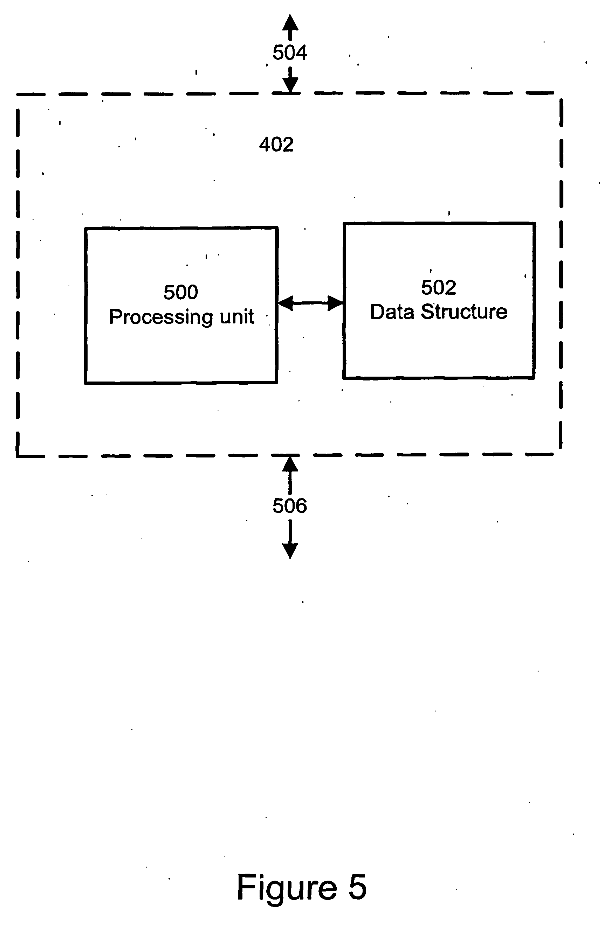

In a specific example of implementation, as shown in figure 5, the broker unit 402 comprises a first communication port 506 suitable for exchanging data with the information gathering unit 404 and a second communication port 504 suitable for exchanging data with the labor progress unit 400. In accordance with the specific example of implementation, the system 450 makes use of a message passing broker pattern. The broker unit 402, the labor progress unit 400, the information gathering unit 404 and the remote display monitoring unit 406 communicate by sending and receiving messages. Each message is a data structure including a destination entity identifier, a sender entity identifier and data elements. This may be implemented by using well know software components such as for example Microsoft™ Message Queue Server. The skilled person in the art will readily appreciate that broker patterns making use of communication data structures other than messages may be used here.

The broker unit 402 also includes a data structure 502 including a plurality of entries. Each entry is associated to a respective component of the system and allows the broker unit to locate the components within the system. More specifically, each entry in the data structure includes the entity identifier of the component along with an address data element. For example, the entity identifier may be associated to an IP address, a process number or any other suitable way of locating a component in the system. The data structure is such that by searching and locating an entity identifier, the address data element can be extracted.

More specifically, for each component in the system 450, an entry is created in the data structure of the broker unit 402. Continuing the specific example of implementation, at least one entry in the data structure is associated to the following components:

> the labor progress unit 400 ;
> the information gathering unit 404 ;
> the remote display monitoring unit 406.

The broker unit 402 also includes a processing unit 500 operative for processing a certain message received from the information gathering unit 404 at the first communication port 506 to extract therefrom a destination entity identifier. The broker unit then locates in the data structure 502 the destination entity identifier and extracts therefrom the address data element associated with the destination entity identifier. The message is then forwarded to the second communication port 504 for transmission to the destination components corresponding to the destination entity identifier on the basis of the extracted address. The processor of the broker unit 402 is also operative for processing a certain message received from the labor progress unit at the second communication port 504. The message is processed to extract therefrom a destination entity identifier. The broker unit then locates in the data structure 502 the destination entity identifier and extracts therefrom the address data element associated with the destination entity identifier. The message is then forwarded to the first communication port 506 for transmission to the information, gathering unit when the certain message includes a destination entity identifier matching the entity identifier corresponding to the information gathering unit.

As a variant, the broker unit 402, further provides publishing/subscribing capabilities. In a specific example, the broker unit makes use of a publish/subscribe pattern (also known as the Observer pattern) which is well known in the art of networks. In this pattern, components in the system can subscribe to events. When these events occur, subscribers to the event are notified. Advantageously, making use of a publish/subscriber pattern instead of other information sharing patterns such as polling allows a reduction in the network bandwidth and resources required.

More specifically in this variant, the entry in the data structure 502 of the broker unit associated to servers, such as the labor progress unit 400, includes a link to a subscriber list suitable for storing entity identifiers. The processor of the broker unit is operative for processing a certain message received from the labor progress unit at the second communication port such as to forward it to each component having an entity identifier in the subscriber list. In this fashion, the component of the system desirous of obtaining information about the result of the labor progress unit automatically receives updates without the requirement to poll the labor progress unit. For example, a centralized nursing station may have a device for displaying showing the labor progress of a patient (or several patients) in various rooms in the obstetrics department. This is shown as the remote display monitoring unit 406 in figure 4.

For more information regarding the use of broker units and publish/subscribe functionality, the reader is invited to consult "Design Patterns, Elements of Reusable Object-oriented Software", Erich Gamma et al., Addison-Wesley, 1995

The set of client units 404 406 includes components such as computer terminals, display units and printers among others. Each client unit is associated to a respective entity identifier allowing distinguishing a certain client unit from the other client units in the set of client units. Each client unit 404 406 includes an input/output port 410 (I/O) for communicating with the broker unit 402.

In a specific example of implementation, the set of client units 404 406 includes an information gathering unit 404 associated to a first entity identifier. The information-gathering unit has an input 408 suitable for receiving a group of clinical measurements associated to a patient. The group of clinical measurements is essentially similar to that described in connection with the input 102 shown in connection with the apparatus of figure 1. Consequently all the characteristics and variants described for the group of clinical measurements apply here as well and will therefore not be described further. The input 408 of the information gathering unit may be in the form of a keyboard, a pointing device such as a mouse coupled to a monitor, a microphone coupled to a speech recognition device, a fetal monitor unit or any other type of input device suitable for receiving data elements indicative of clinical measurements.

The information-gathering unit 404 also includes an output 412 suitable for releasing a signal indicative of a graphical representation of a certain expected dilatation of the cervix. In a specific example of implementation, the output 412 also releases a signal indicative of a graphical representation of a band of expected dilatations of the cervix. The output 412 also releases a signal indicative of a ranking data element. The ranking data element is indicative of a percentile ranking of the current dilatation of the cervix with respect to the calculated certain expected dilatation of the cervix. The output signal is suitable to be displayed by a graphical display unit. In a specific example of implementation, a graphical display unit is coupled to the output 412 such as to provide a graphical display of the results. Very specific examples of graphical display units include a computer monitor, a TV monitor, a LCD screen, and a printer among many other suitable display devices. The output signal released by the output 412 is essentially similar to that described in connection with the output 104 shown in connection with the apparatus of figure 1. Consequently all the characteristics and variants described for the output signal apply here as well and will therefore not be described further.

The information gathering unit 404 also includes a processor operative for assembling the group of clinical measurements into a message data structure for transmission to the broker unit 402. The message data structure includes a destination entity identifier, the first entity identifier associated to the information gathering unit 404 and the group of clinical measurements among others. Messages are a well-known communication device used in distributed computer networks and thus will not be described in further detail here.

The labor progress unit 400 is associated to a second entity identifier allowing distinguishing the labor progress unit from other server units and the client units in the system. The labor progress unit 400 includes an input/output port 414 (I/O) for communicating with the broker unit 402. The labor progress unit is operative for generating a reference measure allowing to assess the progress of childbirth. The labor progress unit 400 implements essentially the same functionality as the processing unit 103 described in connection with figure 1 of the drawings. The labor progress unit 400 is further adapted to receive a message from the broker unit 402 and extract therefrom data element indicative of a group of clinical data elements prior to processing. The labor progress unit 400 is also adapted to assemble the results of the processing into a message data structure for transmission to the broker unit 402. The message data structure includes among others a destination entity identifier, the second entity identifier associated to the labor progress unit 400 and the results of the processing. Messages are a well-known communication device used in distributed computer networks and thus will not be described in further detail here.

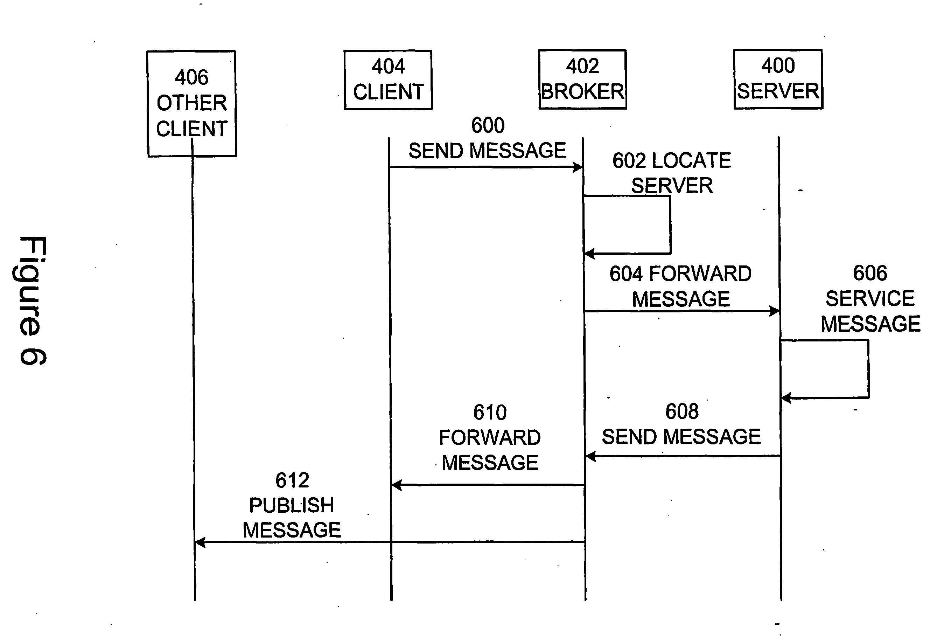

A typical interaction will better illustrate the process implemented by the system 450. In this interaction, the components 400, 404 and 406 have already provided their addresses to the broker unit, and the remote display monitoring unit 406 has subscribed to the labor progress unit 400 and is therefore in the subscriber list associated to the broker data structure entry corresponding to the labor progress unit 400. As shown in figure 6, the information gathering unit 404 sends a message 600 to the broker unit including a group of clinical measurements, a destination entity identifier, a sender entity identifier and requesting the services of the labor progress module 400. The broker unit 402 processes the message and locates the data structure entry corresponding to the destination entity identifier. Once the labor progress unit 400 has been located 602, the message is forwarded at step 604 to the labor progress unit 400. At step 606, the labor progress unit 400 performs its computations and generates a message containing the results. The message has a destination entity identifier corresponding to the information gathering unit 404. At step 608, the message is sent from the labor progress unit 400 to the broker unit. The broker unit 402 processes the message and locates the data structure entry corresponding to the destination entity identifier. Once the information gathering unit 404 has been located, the message is forwarded at step 610 to the information gathering unit 404. Following this, the broker unit also locates in the data structure the entry corresponding to the labor progress unit 400 and processes the subscriber list to which it is associated. In this example, the subscriber list includes an entity identifier associated to the remote display monitoring unit 406. The broker unit forwards the message received from the labor progress module to the remote display monitoring unit 406 at step 612.

Advantageously, by making use of a broker unit network architectural pattern for the system 450, an increased scalability of the system can be obtained. For example, the system 450 may be extended by adding client units, server units without the need to modify or update the existing components.

Another advantage of this type of architecture is location transparency. Since the broker unit locates and coordinates all components there is no need for one component to know where the others are. In other words, if a component is displaced, only the broker unit 402 needs to be advised of the new location.

Yet another advantage is that the different components of the system may be distributed in different computing nodes and even be in remote geographical locations. For example, the labor progress unit 400 may be located in the main computing center of a hospital complex while the information gathering unit 404 may be located in the room of a patient in the obstetrics department. In other words, while the group of clinical measurements is collected in one location, calculations on the group of clinical measurements can be transparently performed in a completely different location. Consequently, a single labor progress unit 400 can be used for a set of patients in different rooms without requiring the displacement of the labor progress unit 400.

Yet another advantage of providing a system making use of a broker architectural pattern is that components may interact with one another over existing computer networks such as for example the Internet.

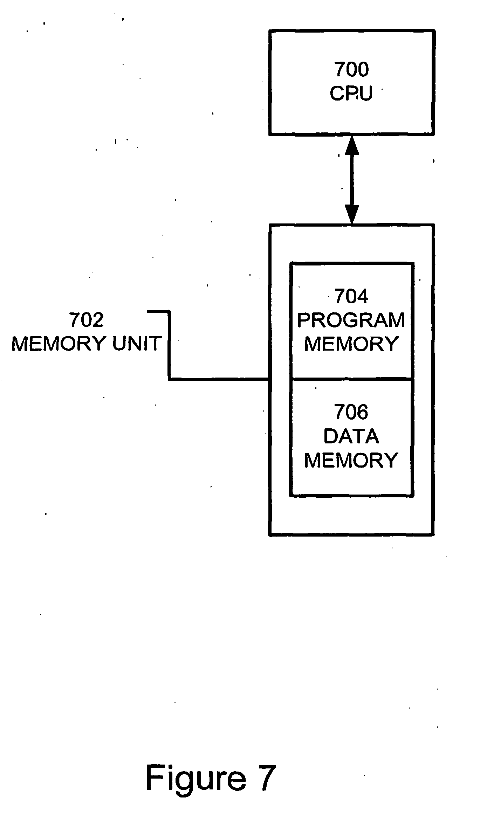

The method described in the specification can also be implemented on any suitable computing platform as shown in figure 7. Such a computing platform typically includes a processor 700 and a memory unit or computer readable medium 702 connected to the processor 700 by a data communication bus. The memory stores the data 706 and the instructions of the program element 704 implementing the functional blocks depicted in the drawings and described in the specification. In a specific example, the program element 704 implements the processing unit 103 described in connection with figure 1. The program element 704 operates on the data 706 in accordance with the algorithms described above to generate a reference measure indicative of a certain expected dilatation of the cervix allowing assessing the progress of childbirth. In another specific example, the program element 704 is operative for implementing the broker unit 402 described in connection with figure 4. In yet another specific example, the program element 704 is operative for implementing the information gathering unit 404 described in connection with figure 4. In yet another specific example, the program element 704 is operative for implementing labor progress unit 400 described in connection with figure 4. The computing apparatus typically executes an operating system. A very specific example of implementation makes use of a general purpose digital computer running the Microsoft™ Windows NT™ operating system. Microsoft™ Windows NT™ may be used to integrate multiple components such as databases, messaging, Web portals and application packaging among others. Other operating systems may be used.

Although the present invention has been described in considerable detail with reference to certain preferred embodiments thereof, variations and refinements are possible , the scope of the invention should be limited only by the appended claims.

**Claims**

1. An apparatus (100) for tracking the labor progress of a patient during childbirth, said apparatus comprising:

   input means (102) for receiving a group of clinical measurements including data elements indicative of a measurement of a previous dilatation of the cervix of the patient, a contraction count, a previous level of descent of the child, a previous effacement measurement of the cervix, an epidural status and a parity status, said parity status being indicative of either one of a first childbirth for the patient and a childbirth subsequent to a first childbirth ;
   processing means (103) coupled to said input means (102), said processing means (103) being operative for generating a reference measure indicative of a certain expected dilatation of the cervix allowing to assess the progress of childbirth by using the following formula:

   $$x= AA + BB*(1-y) + CC*z + DD*w + EE*v + FF*u$$

   where:

   x is the certain expected dilatation of the cervix;
   y is the epidural status;

z is the previous dilatation of the cervix measurement;

w is the previous effacement measurement of the cervix;

v is the previous level of descent of the child:

u is the contraction count;

and where AA, BB, CC, DD, EE and FF are a set of real numbers conditioned at least in part on the basis of said parity status;

output means (104) for releasing a signal indicative of a measurement of the certain expected dilatation of the cervix.

2. An apparatus as defined in claim 1, wherein when said parity status is indicative of a first childbirth, the first set of real numbers is **characterized by**:

AA having a value of about 0.24952;

BB having a value of about 0.14627;

CC having a value of about 0.80515;

DD having a value of about 0.24109;

EE having a value of about 0.03909;

FF having a value of about 0,00447.

3. An apparatus as defined in claim 1, wherein when said parity status is indicative of a childbirth subsequent to a first childbirth, the set of real numbers is a second set of real numbers **characterized by**:

AA having a value of about 2;

BB having a value of about 0.2;

CC having a value of about 0.8;

DD having a value of about 0.15;

EE having a value of about 0.2;

FF having a value of about 0.01.

4. An apparatus as defined in claim 3, wherein the second set of real numbers is **characterized by**:

AA having a value of about 1.9914;

BB having a value of about 0.23484;

CC having a value of about 0.7770;

DD having a value of about 0.15503;

EE having a value of about 0.20669;

FF having a value of about 0.00766.

5. An apparatus as defined in claim 1, wherein said formula includes an error estimate data element indicative of a residual value, said error estimate allowing to derive a range of expected dilatations of the cervix having a normal distribution.

6. An apparatus as defined in claim 5, wherein said error estimate data element has a value of about 1.3.

7. An apparatus as defined in claim 6, wherein when said parity status is indicative of a first childbirth, the set of real numbers is a first set of real numbers **characterized by**:

AA having a value of about 0.24952;

BB having a value of about 0.14627;

CC having a value of about 0.80515;

DD having a value of about 0.24109;

EE having a value of about 0.03909;

FF having a value of about 0.00447;

and said error estimate has a value of about 1.3014.

8. An apparatus as defined in claim 6, wherein when said parity status is indicative of a childbirth subsequent to a first childbirth, the set of real numbers is a second set of real numbers **characterized by**:

AA having a value of about 1.9914;
BB having a value of about 0.23484;
CC having a value of about 0.7770;
DD having a value of about 0.15503;
EE having a value of about 0.20669;
FF having a value of about 0.00766;
and said error estimate has a value of about 1.3451.

9. An apparatus as defined in claim 5, wherein said group of clinical measurements includes a data element indicative of a current dilatation of the cervix measurement.

10. An apparatus as defined in claim 9, wherein said processing means (103) are operative for processing said current dilatation of the cervix measurement to derive a ranking data element indicative of a percentile ranking of the current dilatation of the cervix measurement with respect to the range of expected dilatations of the cervix on the basis of said certain expected dilatation of the cervix and said error estimate.

11. An apparatus as defined in claim 10, wherein said output means (104) are further for releasing a signal indicative of the ranking data element.

12. An apparatus as defined in claim 5, wherein said processing means (103) are further operative to generate a plurality of expected dilatations of the cervix defining a reference band of expected dilatations of the cervix, the reference band of expected dilatations of the cervix including said certain expected dilatation of the cervix, the reference band being a portion of said range of expected dilatations of the cervix.

13. An apparatus as defined in claim 12, wherein said output means (104) are further for releasing a signal indicative of the reference band of expected dilatations of the cervix.

14. An apparatus as defined in claim 13, wherein said input means (102) are for receiving a plurality of groups of clinical measurements, each group of clinical measurements being associated with a respective time, said processing means (103) being operative for processing the plurality of groups of clinical measurements to generate a sequence of certain expected dilatations of the cervix, said output means (104) being also operative for releasing a signal indicative of the sequence of certain expected dilatations of the cervix.

15. An apparatus as defined in claim 14, wherein said processing means (103) are further operative to generate a sequence of reference bands of expected dilatations of the cervix, said output means (104) being also operative for releasing a signal indicative of a graphical representation of the sequence of reference bands of expected dilatations of the cervix.

16. A computer readable medium (702) comprising a program element (704) suitable for execution by a computing apparatus for tracking the labor progress of a patient during childbirth, said computing apparatus including a processor (700), said program element (704) when executing on said processor (700) being operative for implementing:

an input (102) for receiving a group of clinical measurements associated to a patient, said group of clinical measurements including data elements indicative of a measurement of a previous dilatation of the cervix of the patient, a contraction count, a previous level of descent of the child, a previous effacement measurement of the cervix, an epidural status and a parity status, said parity status being indicative of either one of a first childbirth for the patient and a childbirth subsequent to a first childbirth;
a processing unit (103) for generating a reference measure indicative of a certain expected dilatation of the cervix allowing to assess the progress of childbirth by using the following formula:

$$x = AA + BB*(1-y) + CC*z + DD*w + EE*v + FF*u$$

where:

x is the certain expected dilatation of the cervix;
y is the epidural status;

z is the previous dilatation of the cervix measurement;
w is the previous effacement measurement of the cervix;
v is the previous level of descent of the child;
u is the contraction count;
and where AA, BB, CC, DD, EE and FF are a set of real numbers conditioned at least in part on the basis of said parity status; and,
an output (104) for releasing a signal indicative of a measurement of said certain expected dilatation of the cervix.

17. A computer data signal including a set of source code segments embodied in a transmission medium, said set of source code segments being adapted to be executed by a computing apparatus, said computer data signal comprising:

a clinical measurement collection source code segment comprising a first program element suitable for execution on a computing apparatus, said first program element when executing on the computing apparatus being operative for implementing an information gathering unit, the information gathering unit being suitable for receiving a group of clinical measurements associated to a patient, said group of clinical measurements including data elements indicative of a measurement of a previous dilatation of the cervix of the patient, a contraction count, a previous level of descent of the child, a previous effacement measurement of the cervix, an epidural status and a parity status, said parity status being indicative of either one of a first childbirth for the patient and a childbirth subsequent to a first childbirth;
a labor tracking source code segment comprising a second program element suitable for execution on a computing apparatus, said second program element when executing on the computing apparatus being operative for implementing a labor progress unit, the labor progress unit being operative for generating a reference measure indicative of a certain expected dilatation of the cervix allowing to assess the progress of childbirth by using the following formula:

$$x= AA + BB*(1-y) +CC*z + DD*w +EE*v + FF*u$$

where:

x is the certain expected dilatation of the cervix;
y is the epidural status;
z is the previous dilatation of the cervix measurement;
w is the previous effacement measurement of the cervix;
v is the previous level of descent of the child;
u is the contraction count;

and where AA, BB, CC, DD, EE and FF are a set of real numbers conditioned at least in part on the basis of said parity status, the labor progress unit being operative for releasing a signal indicative of a measure of the certain expected dilatation of the cervix ; and,
an output source code segment comprising a third program element suitable for execution on a computing apparatus, said third program element when executing on the computing apparatus being operative for implementing an output display device for conveying said measure of the certain expected dilatation of the cervix.

**Patentansprüche**

1. Gerät (100) zum Verfolgen des Fortschritts der Geburtsarbeit einer Patientin während der Geburt eines Kindes, umfassend:

Eingabemittel (102) für den Empfang einer Gruppe von klinischen Messungen enthaltend Datenelemente, die auf eine Messung einer vorherigen Weitung der Zervix der Patientin, eine Kontraktionszählung, eine vorherige Stufe des Descensus des Kindes, eine vorherige Messung der Zervixverkürzung, einen Epiduralstatus und einen Paritätsstatus schließen lassen, wobei der Paritätsstatus darauf schließen lässt, ob die Geburt eine erste Kindsgeburt oder eine auf eine erste Kindsgeburt folgende Kindsgeburt ist;
Verarbeitungsmittel (103), die mit den Eingabemitteln (102) gekoppelt sind, wobei die Verarbeitungsmittel (103) wirksam sind, um ein Referenzmaß zu erzeugen, das eine bestimmte erwartete Weitung der Zervix angibt und eine Beurteilung des Geburtsfortschritts durch eine Anwendung der folgenden Formel erlaubt:

$$x = AA + BB^* (1-y) + CC^*z + DD^*w + EE^*v + FF^*u,$$

wobei:

x die bestimmte erwartete Weitung der Zervix ist;
y der Epiduralstatus ist;
z die vorherige Messung der Zervixweitung ist;
w die vorherige Messung der Zervixverkürzung ist;
v die vorherige Stufe des Descensus des Kindes ist;
u die Zählung der Kontraktionen ist;
und wobei AA, BB, CC, DD, EE und FF ein Satz von reellen Zahlen sind, die zumindest teilweise durch den Paritätsstatus bedingt sind; Ausgabemittel (104) für die Ausgabe eines Signals, das auf eine Messung der bestimmten erwarteten Weitung der Zervix schließen lässt.

2.  Gerät nach Anspruch 1, wobei bei einem auf eine erste Kindsgeburt hinweisenden Paritätsstatus der erste Satz von reellen Zahlen **dadurch gekennzeichnet ist, dass**

AA einen Wert von circa 0,24952 hat;
BB einen Wert von circa 0,14627 hat;
CC einen Wert von circa 0,80515 hat;
DD einen Wert von circa 0,24109 hat;
EE einen Wert von circa 0,03909 hat;
FF einen Wert von circa 0,00447 hat.

3.  Gerät nach Anspruch 1, wobei bei einem auf eine Kindsgeburt nach einer ersten Kindsgeburt hinweisenden Paritätsstatus der Satz von reellen Zahlen ein zweiter Satz von reellen Zahlen ist, **dadurch gekennzeichnet, dass**

AA einen Wert von circa 2 hat;
BB einen Wert von circa 0,2 hat;
CC einen Wert von circa 0,8 hat;
DD einen Wert von circa 0,15 hat;
EE einen Wert von circa 0,2 hat;
FF einen Wert von circa 0,01 hat.

4.  Gerät nach Anspruch 3, wobei der zweite Satz von reellen Zahlen, **dadurch gekennzeichnet ist, dass**

AA einen Wert von circa 1,9914 hat;
BB einen Wert von circa 0,23484 hat;
CC einen Wert von circa 0,7770 hat;
DD einen Wert von circa 0,15503 hat;
EE einen Wert von circa 0,20669 hat;
FF einen Wert von circa 0,00766 hat.

5.  Gerät nach Anspruch 1, wobei die Formel einen Fehlerschätzungs-Datenelement enthält, das auf einen Restwert schließen lässt, wobei die Fehlerschätzung erlaubt, einen Bereich von erwarteten Weitungen der Zervix abzuleiten, der eine normale Verteilung hat.

6.  Gerät nach Anspruch 5, wobei das Fehlerschätzungs-Datenelement einen Wert von circa 1,3 hat.

7.  Gerät nach Anspruch 6, wobei bei einem auf eine erste Kindsgeburt hinweisenden Paritätsstatus der Satz von reellen Zahlen ein erster Satz von reellen Zahlen ist, **dadurch gekennzeichnet ist, dass**

AA einen Wert von circa 0,24952 hat;

BB einen Wert von circa 0,14627 hat;
CC einen Wert von circa 0,80515 hat;
DD einen Wert von circa 0,24109 hat;
EE einen Wert von circa 0,03909 hat;
FF einen Wert von circa 0,00447 hat
und dass die Fehlerschätzung einen Wert von circa 1,3014 hat.

8.  Gerät nach Anspruch 6, wobei bei einem auf eine Kindsgeburt nach einer ersten Kindsgeburt hinweisenden Paritätsstatus der Satz von reellen Zahlen ein zweiter Satz von reellen Zahlen ist, **dadurch gekennzeichnet, dass**

AA einen Wert von circa 1,9914 hat;
BB einen Wert von circa 0,23484 hat;
CC einen Wert von circa 0,7770 hat;
DD einen Wert von circa 0,15503 hat;
EE einen Wert von circa 0,20669 hat;
FF einen Wert von circa 0,00766 hat
und dass die Fehlerschätzung einen Wert von circa 1,3451 hat.

9.  Gerät nach Anspruch 5, wobei die Gruppe von klinischen Messungen ein Datenelement enthält, das auf eine aktuelle Messung der Zervixweitung hindeutet.

10. Gerät nach Anspruch 9, wobei die Verarbeitungsmittel (103) wirksam sind, um die aktuelle Messung der Zervixweitung zu verarbeiten, um ein Rangdatenelement abzuleiten, das auf einen Durchschnittsrang der aktuellen Messung der Zervixweitung bezüglich des Rangs von erwarteten Weitungen der Zervix auf der Basis der bestimmten erwarteten Zervixweitung und der Fehlerschätzung schließen lässt.

11. Gerät nach Anspruch 10, wobei die Ausgabemittel (104) ferner zur Ausgabe eines Signals dienen, welches auf das Rangdatenelement hindeutet.

12. Gerät nach Anspruch 5, wobei die Verarbeitungsmittel (103) ferner wirksam sind, um eine Mehrzahl von erwarteten Weitungen der Zervix zu erzeugen, die ein Referenzband von erwarteten Weitungen der Zervix definiert, wobei das Referenzband von erwarteten Weitungen der Zervix die bestimmte erwartete Weitung der Zervix enthält, wobei das Referenzband ein Teil des Bereichs von erwarteten Weitungen der Zervix ist.

13. Gerät nach Anspruch 12, wobei die Ausgabemittel (104) ferner wirksam sind, um ein Signal auszugeben, das auf das Referenzband von erwarteten Weitungen der Zervix hindeutet.

14. Gerät nach Anspruch 13, wobei die Eingabemittel (102) für den Empfang einer Mehrzahl von Gruppen von klinischen Messungen dienen, wobei jede Gruppe von klinischen Messungen einer jeweiligen Zeit zugeordnet ist, wobei die Verarbeitungsmittel (103) wirksam sind für die Verarbeitung der Mehrzahl von Gruppen von klinischen Messungen, um eine Sequenz von bestimmten erwarteten Weitungen der Zervix zu erzeugen, wobei die Ausgabemittel (104) auch wirksam sind für die Ausgabe eines Signals, das auf die Sequenz von bestimmten erwarteten Weitungen der Zervix hindeutet.

15. Gerät nach Anspruch 14, wobei die Verarbeitungsmittel (103) ferner wirksam sind für die Erzeugung einer Sequenz von Referenzbändern von erwarteten Weitungen der Zervix, wobei die Ausgabemittel (104) ebenfalls wirksam sind für die Ausgabe eines Signals, das auf eine graphische Darstellung der Sequenz von Referenzbändern von erwarteten Weitungen der Zervix hindeutet.

16. Computerlesbares Medium (702), umfassend ein Programmelement (704), das geeignet ist für die Abarbeitung durch eine Rechenvorrichtung zum Verfolgen des Fortschritts der Geburtsarbeit einer Patientin während einer Kindsgeburt, wobei die Rechenvorrichtung einen Prozessor (700) aufweist, wobei das Programmelement (704) bei Abarbeitung in dem Prozessor (700) wirksam ist zum Implementieren:

eines Eingangs (102) zum Empfangen einer Gruppe von klinischen Messungen, die einer Patientin zugeordnet sind, wobei die Gruppe von klinischen Messungen Datenelemente enthält, die eine Messung einer vorherigen Weitung der Zervix der Patientin, eine Zählung der Kontraktionen, eine vorherige Stufe des Descensus des Kindes, eine vorherige Messung der Zervixverkürzung, einen epiduralen Status und

einen Paritätsstatus angeben, wobei der Paritätsstatus auf eine erste Kindsgeburt der Patientin oder eine auf eine erste Kindsgeburt folgende Kindsgeburt hindeutet;

eine Verarbeitungseinheit (103) zum Erzeugen eines Referenzmaßes, das auf eine bestimmte erwartete Weitung der Zervix hindeutet und eine Beurteilung des Fortschritts der Kindsgeburt unter Anwendung der folgenden Formel erlaubt:

$$x = AA + BB^* (1-y) + CC^*z + DD^*w + EE^*v + FF^*u,$$

wobei:

x die bestimmte erwartete Weitung der Zervix ist;
y der Epiduralstatus ist;
z die vorherige Messung der Zervixweitung ist;
w die vorherige Messung der Zervixverkürzung ist;
v die vorherige Stufe des Descensus des Kindes ist;
u die Zählung der Kontraktionen ist;
und wobei AA, BB, CC, DD, EE und FF ein Satz von reellen Zahlen sind, die zumindest teilweise durch den Paritätsstatus bedingt sind; und
einen Ausgang (104) für die Ausgabe eines Signals, das auf eine Messung der bestimmten erwarteten Zervixweitung schließen lässt.

17. Computerdatensignal, das einen Satz von Quellcodesegmenten enthält, die in einem Übertragungsmedium enthalten sind, wobei der Satz von Quellcodesegmenten für eine Abarbeitung durch eine Rechenvorrichtung geeignet ist, wobei das Computerdatensignal umfasst:

ein Quellcodesegment einer Sammlung von klinischen Messungen, umfassend ein erstes Programmelement, das für die Abarbeitung in einer Rechenvorrichtung geeignet ist, wobei das erste Programmelement bei der Abarbeitung in der Rechenvorrichtung wirksam ist für die Implementierung einer Informationserfassungseinheit, wobei die Informationserfassungseinheit geeignet ist für den Empfang einer Gruppe von klinischen Messungen, die einer Patientin zugeordnet sind, wobei die Gruppe von klinischen Messungen Datenelemente enthält, die auf eine Messung einer vorherigen Weitung der Zervix der Patientin, eine Kontraktionszählung, einen vorherigen Grad des Descensus des Kindes, eine vorherige Messung der Zervixverkürzung, einen Epiduralstatus und eine Paritätsstatus schließen lassen, wobei der Paritätsstatus auf eine erste Kindsgeburt für die Patientin oder auf eine der ersten Kindsgeburt folgende Kindsgeburt schließen lässt;
ein Quellcodesegment der Verfolgung der Geburtsarbeit, umfassend ein zweites Programmelement, das geeignet ist für die Abarbeitung in einer Rechenvorrichtung, wobei das zweite Programmelement bei der Abarbeitung in der Rechenvorrichtung wirksam ist für die Implementierung einer Einheit für den Fortschritt der Geburtsarbeit, wobei die Einheit für den Fortschritt der Geburtsarbeit wirksam ist für die Erzeugung eines Referenzmaßes, das eine bestimmte erwartete Weitung der Zervix angibt und eine Beurteilung des Fortschritts der Kindsgeburt unter Anwendung der folgenden Formel erlaubt:

$$x = AA + BB^* (1-y) + CC^*z + DD^*w + EE^*v + FF^*u,$$

.

wobei:

x die bestimmte erwartete Weitung der Zervix ist;
y der Epiduralstatus ist;
z die vorherige Messung der Zervixweitung ist;
w die vorherige Messung der Zervixverkürzung ist;
v die vorherige Stufe des Descensus des Kindes ist;
u die Zählung der Kontraktionen ist;
und wobei AA, BB, CC, DD, EE und FF ein Satz von reellen Zahlen sind, die zumindest teilweise durch den Paritätsstatus bedingt sind; wobei die Einheit für den Fortschritt der Geburtsarbeit wirksam ist für die Ausgabe eines Signals, das auf ein Maß der bestimmten erwarteten Weitung der Zervix schließen lässt; und

ein Ausgabequellcodesegment, das ein drittes Programmelement umfasst, das geeignet ist für die Abarbeitung in einer Rechenvorrichtung, wobei das dritte Programmelement bei der Abarbeitung in der Rechenvorrichtung wirksam ist für die Implementierung einer Ausgabeanzeigevorrichtung zur Übermittlung des Maßes der bestimmten erwarteten Weitung der Zervix.

**Revendications**

1. Appareil (100) pour suivre la progression du travail d'une patiente au cours de l'accouchement, ledit appareil comprenant :

un moyen d'entrée (102) pour la réception d'un groupe de mesures cliniques comportant des éléments de données indicateurs d'une mesure précédente de la dilatation du col de la patiente, du nombre de contractions, d'un niveau précédent de descente de l'enfant, d'une mesure précédente de l'effacement du col, d'un statut épidural et d'un statut de parité, ledit statut de parité étant indicateur soit d'un premier accouchement de la patiente soit d'un accouchement ultérieur à un premier accouchement ;
un moyen de traitement (103) couplé audit moyen d'entrée (102), ledit moyen de traitement (103) étant fonctionnel pour la génération d'une mesure de référence indicatrice d'une certaine dilatation attendue du col permettant d'évaluer la progression de l'accouchement au moyen de la formule suivante :

$$x = AA + BB*(1-y) + CC*z + DD*w + EE*v + FF*u$$

où :

x est la certaine dilatation attendue du col ;
y est le statut épidural ;
z est la mesure précédente de la dilatation du col ;
w est la mesure précédente de l'effacement du col ;
v est le niveau précédent de descente de l'enfant ;
u est le nombre de contractions ;

et/où AA, BB, CC, DD, EE, et FF sont un ensemble de nombres réels conditionnés au moins en partie sur la base dudit statut de parité ;
un moyen de sortie (104) pour la libération d'un signal indicateur d'une mesure de la certaine dilatation attendue du col.

2. Appareil tel que défini dans la revendication 1, dans lequel quand ledit statut de parité est indicateur d'un premier accouchement, le premier ensemble de nombres réels est **caractérisé par** :

AA ayant une valeur d'environ 0,24952 ;
BB ayant une valeur d'environ 0,14627 ;
CC ayant une valeur d'environ 0,80515 ;
DD ayant une valeur d'environ 0,24109 ;
EE ayant une valeur d'environ 0,03909 ;
FF ayant une valeur d'environ 0,00447.

3. Appareil tel que défini dans la revendication 1, dans lequel quand ledit statut de parité est indicateur d'un accouchement ultérieur à un premier accouchement, l'ensemble de nombres réels est un second ensemble de nombres réels **caractérisé par** :

AA ayant une valeur d'environ 2 ;
BB ayant une valeur d'environ 0,2 ;
CC ayant une valeur d'environ 0,8 ;
DD ayant une valeur d'environ 0,15 ;
EE ayant une valeur d'environ 0,2 ;
FF ayant une valeur d'environ 0,01.

**4.** Appareil tel que défini dans la revendication 3, dans lequel le second ensemble de nombres réels est **caractérisé par** :

AA ayant une valeur d'environ 1,9914 ;
BB ayant une valeur d'environ 0,23484 ;
CC ayant une valeur d'environ 0,7770 ;
DD ayant une valeur d'environ 0,15503 ;
EE ayant une valeur d'environ 0,20669 ;
FF ayant une valeur d'environ 0,00766.

**5.** Appareil tel que défini dans la revendication 1, dans lequel ladite formule comporte un élément de données d'estimation d'erreur indicateur d'une valeur résiduelle, ladite estimation d'erreur permettant de dériver une plage de dilatations attendues du col ayant une distribution normale.

**6.** Appareil tel que défini dans la revendication 5, dans lequel ledit élément de données d'estimation d'erreur a une valeur d'environ 1,3.

**7.** Appareil tel que défini dans la revendication 6, dans lequel quand ledit statut de parité est indicateur d'un premier accouchement, l'ensemble de nombres réels est un premier ensemble de nombres réels **caractérisé par** :

AA ayant une valeur d'environ 0,24952 ;
BB ayant une valeur d'environ 0,14627 ;
CC ayant une valeur d'environ 0,80515 ;
DD ayant une valeur d'environ 0,24109 ;
EE ayant une valeur d'environ 0,03909 ;
FF ayant une valeur d'environ 0,00447 ;
et ladite estimation d'erreur a une valeur d'environ 1,3014.

**8.** Appareil tel que défini dans la revendication 6, dans lequel quand ledit statut de parité est indicateur d'un accouchement ultérieur à un premier accouchement, l'ensemble de nombres réels est un second ensemble de nombres réels **caractérisés par** :

AA ayant une valeur d'environ 1,9914 ;
BB ayant une valeur d'environ 0,23484 ;
CC ayant une valeur d'environ 0,7770 ;
DD ayant une valeur d'environ 0,15503 ;
EE ayant une valeur d'environ 0,20669 ;
FF ayant une valeur d'environ 0,00766 ;
et ladite estimation d'erreur a une valeur d'environ 1,3451.

**9.** Appareil tel que défini dans la revendication 5, dans lequel ledit groupe de mesures cliniques comporte un élément de données indicateur de la mesure d'une dilatation actuelle du col.

**10.** Appareil tel que défini dans la revendication 9, dans lequel ledit moyen de traitement (103) est fonctionnel pour le traitement de ladite mesure de la dilatation actuelle du col en vue de dériver un élément de données de classement indicateur d'un classement en centile de la mesure de la dilatation actuelle du col par rapport à la plage de dilatations attendues du col sur la base de ladite certaine dilatation attendue du col et de ladite estimation d'erreur.

**11.** Appareil tel que défini dans la revendication 10, dans lequel ledit moyen de sortie (104) est en outre destiné à la libération d'un signal indicateur de l'élément de données de classement.

**12.** Appareil tel que défini dans la revendication 5, dans lequel ledit moyen de traitement (103) est en outre fonctionnel pour générer plusieurs dilatations attendues du col définissant une bande de référence de dilatations attendues du col, la bande de référence de dilatations attendues du col comportant ladite certaine dilatation attendue du col, la bande de référence étant une partie de ladite plage de dilatations attendues du col.

**13.** Appareil tel que défini dans la revendication 12, dans lequel ledit moyen de sortie (104) est en outre destiné à la libération d'un signal indicateur de la bande de référence de dilatations attendues du col.

**14.** Appareil tel que défini dans la revendication 13, dans lequel ledit moyen d'entrée (102) est destiné à la réception de plusieurs groupes de mesures cliniques, chaque groupe de mesures cliniques étant associé à un instant respectif, ledit moyen de traitement (103) étant fonctionnel pour le traitement de la pluralité de groupes de mesures cliniques en vue de générer une séquence de certaines dilatations attendues du col, ledit moyen de sortie (104) étant également fonctionnel pour la libération d'un signal indicateur de la séquence de certaines dilatations attendues du col.

**15.** Appareil tel que défini dans la revendication 14, dans lequel ledit moyen de traitement (103) est en outre fonctionnel en vue de générer une séquence de bandes de référence de dilatations attendues du col, ledit moyen de sortie (104) étant également fonctionnel pour libérer un signal indicateur d'une représentation graphique de la séquence de bandes de référence de dilatations attendues du col.

**16.** Moyen (702) lisible par ordinateur, comprenant un élément de programme (704) approprié pour une exécution par un appareil de calcul en vue de suivre la progression du travail d'une patiente au cours de l'accouchement, ledit appareil de calcul comportant un processeur (700), ledit élément de programme (704) lors de son exécution sur ledit processeur (700) étant fonctionnel pour mettre en oeuvre :

une entrée (102) pour la réception d'un groupe de mesures cliniques associées à une patiente, ledit groupe de mesures cliniques comportant des éléments de données indicateurs d'une mesure précédente de la dilatation du col de la patiente, d'un nombre de contractions, d'un niveau précédent de descente de l'enfant, d'une mesure précédente de l'effacement du col, d'un statut épidural et d'un statut de parité, ledit statut de parité étant indicateur soit d'un premier accouchement pour la patiente soit d'un accouchement ultérieur à un premier accouchement ;
une unité de traitement (103) en vue de générer une mesure de référence indicatrice d'une certaine dilatation attendue du col permettant d'évaluer la progression de l'accouchement au moyen de la formule suivante :

$$x = AA + BB*(1-y) + CC*z + DD*w + EE*v + FF*u$$

(104)
où :

x est la certaine dilatation attendue du col ;
y est le statut épidural ;
z est la mesure précédente de la dilatation du col ;
w est la mesure précédente de l'effacement du col ;
v est le niveau précédent de descente de l'enfant ;
u est le nombre de contractions ;

et/où AA, BB, CC, DD, EE, et FF sont un ensemble de nombres réels conditionnés au moins en partie sur la base dudit statut de parité ; et
d'une sortie (104) pour la libération d'un signal indicateur d'une mesure de ladite certaine dilatation attendue du col.

**17.** Signal de données informatiques comportant un ensemble de segments de code source incorporé dans un milieu de transmission, ledit ensemble de segments de code source étant adapté de manière à être exécuté par un appareil de calcul, ledit signal de données informatiques comprenant :

un segment de code source de récolte de mesures cliniques comprenant un premier élément de programme approprié pour une exécution sur un appareil de calcul, ledit premier élément de programme, lors d'une exécution sur l'appareil de calcul étant fonctionnel pour mettre en oeuvre une unité de regroupement d'informations, l'unité de regroupement d'informations étant appropriée pour la réception d'un groupe de mesures cliniques associées à une patiente, ledit groupe de mesures cliniques comportant des éléments de données indicateurs d'une mesure précédente de la dilatation du col de la patiente, d'un nombre de contractions, d'un niveau précédent de descente de l'enfant, de la mesure précédente de l'effacement du col, d'un statut épidural et d'un statut de parité, ledit statut de parité étant indicateur soit d'un premier accouchement pour la patiente soit d'un accouchement ultérieur à un premier accouchement ;
un segment de code source de suivi du travail comprenant un second élément de programme approprié pour une exécution sur un appareil de calcul, ledit second élément de programme, lors d'une exécution sur l'appareil de calcul étant fonctionnel pour la mise en oeuvre d'une unité de progression du travail, l'unité de progression du travail étant fonctionnelle pour la génération d'une mesure de référence indicatrice d'une certaine dilatation attendue du col permettant d'évaluer la progression de l'accouchement au moyen de la formule suivante :

$$x = AA + BB*(1-y) + CC*z + DD*w + EE*v + FF*u$$

où :

x est la certaine dilatation attendue du col ;
y est le statut épidural ;
z est la mesure précédente de la dilatation du col ;
w est la mesure précédente de l'effacement du col ;
v est le niveau précédent de descente de l'enfant ;
u est le nombre de contractions ;

et/où AA, BB, CC, DD, EE, et FF sont un ensemble de nombres réels conditionnés au moins en partie sur la base dudit statut de parité, l'unité de progression du travail étant fonctionnelle pour la libération d'un signal indicateur d'une mesure de la certaine dilatation attendue du col ; et
un segment de code source de sortie comprenant un troisième élément de programme approprié pour une exécution sur un appareil de calcul, ledit troisième élément de programme, lors d'une exécution sur l'appareil de calcul, étant fonctionnel pour la mise en oeuvre d'un dispositif d'affichage de sortie pour la transmission de ladite mesure de la certaine dilatation attendue du col.

102
Input

100

103
Processing unit

104
Output

Figure 1

**102**
Input

diagnostic
measurements

**202**
Computing sub-
unit for calculating
the expected
cervix dilatation
and range

**203**
Expected cervix
dilatation and
range

current cervix
dilatation

**204**
Ranking
computation sub-
unit

**104**
Ouput

**206**
Ranking data element

# Figure 2

# LABOR PROGRESS

FIG. 3

—450

400
Server
Labour Progress unit

414

402
Broker unit

410                              410

404
Client #1
Information Gathering unit

406
Client #2
Remote display
monitoring unit

412
Output

408
Input

Figure 4

504

402

| 500 Processing unit | 502 Data Structure |

506

# Figure 5

Figure 6

700
CPU

702
MEMORY UNIT

704
PROGRAM
MEMORY

706
DATA
MEMORY

# Figure 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- INTRAPARTUM PREDICTION OF FETAL STATUS AND ASSESSMENT OF LABOUR PROGRESS. *BAILLIERES CLINICAL OBSTETRICS AND GYNAECOLOGY, BAILLIERE TINDALL,* 08 September 1994, vol. 8 (3), 567-581

- **ERICH GAMMA et al.** Design Patterns, Elements of Reusable Object-oriented Software. *Addison-Wesley,* 1995